# EUROPÄISCHE PATENTSCHRIFT

(11) **EP 1 575 649 B1**
(45) Veröffentlichungstag und Bekanntmachung des Hinweises auf die Patenterteilung: **23.08.2017**
(21) Anmeldenummer: 03785716.6
(22) Anmeldetag: 03.12.2003
(51) Int. Cl.: A61M 15/00, B65D 83/04

(54) **PULVERINHALATOR MIT KAPSELKAMMER ZUM AUFNEHMEN EINER MIT WIRKSTOFF GEFÜLLTEN EINWEGKAPSEL**
POWDER INHALER WITH A CAPSULE CHAMBER FOR RECEIVING A DISPOSABLE CAPSULE FILLED WITH AN ACTIVE SUBSTANCE
INHALATEUR DE POUDRE POURVU D'UNE CHAMBRE DESTINEE A RECEVOIR UNE CAPSULE JETABLE REMPLIE D'UN PRINCIPE ACTIF

(30) Priorität: 12.12.2002 DE 10258360
(43) Veröffentlichungstag der Anmeldung: 21.09.2005
(73) Patentinhaber: Boehringer Ingelheim Pharma GmbH & Co. KG, 55216 Ingelheim am Rhein (DE)
(72) Erfinder: HOCHRAINER, Dieter, 57392 SCHMALLENBERG (DE)
(86) Internationale Anmeldenummer: PCT/EP2003/013612
(87) Internationale Veröffentlichungsnummer: WO 2004/052435

(56) Entgegenhaltungen:
- EP-A- 0 303 844
- EP-A- 0 911 047
- WO-A-00/07572
- WO-A-91/02558
- WO-A-02/083220
- DE-A- 2 529 522
- DE-A- 3 345 722
- GB-A- 2 356 842
- US-A- 2 604 094
- US-A1- 2002 134 374

## Beschreibung

Die Erfindung betrifft einen nach dem Bernoulli-Prinzip arbeitenden Pulverinhalator mit austauschbaren zylinderartigen Einwegkapseln als Wirkstoffreservoir. Der Pulverinhalator besteht im Wesentlichen aus einer bevorzugt zylinderartigen Kapselkammer, die mit Mitteln zum seitlichen Öffnen der Kapsel versehen ist, einer Lufteinlassöffnung in der Kapselkammer, und einer Luftauslassöffnung und einem, der Luftauslassöffnung nachgeschalteten Mundstück. Die Kapselkammer ist so gestaltet, dass die eingesetzte Reservoirkapsel im Wesentlichen nur eine Bewegung in Längsrichtung vollführen kann, wenn ein im Wesentlichen parallel zur Längsachse der Kapsel geführter Luftstrom durch die Kapselkammer geführt wird. Erfindungsgemäß weist die Innenwand der Kapselkammer Unebenheiten auf, um eine gegenüber einer glatten Innenoberfläche verbesserte Kapselentleerung zu erreichen.

### Stand der Technik

Die Literatur kennt mehrere nach dem Bernoulli -Prinzip arbeitende Pulverinhalatoren. Allen ist gemeinsam, dass der auszubringende Wirkstoff in einer zylinderartigen Kapsel gelagert wird und diese Kapsel in eine Kapselkammer des Inhalators eingesetzt wird. Die Kapselkammer ist dabei meist ebenfalls zylindrisch ausgebildet, wobei sie etwas länger und etwas breiter als die Kapsel ist, so dass die Kapsel in ihr zwar sowohl longitudinal, d.h. in Strömungsrichtung der Luft, als auch transversal, d.h. quer zur Strömungsrichtung, vibrieren kann, dabei aber im Wesentlichen parallel zur Kammerachse ausgerichtet bleibt. Die Kapselkammer weist im Bereich eines der beiden Enden einen Lufteinlass und im Bereich des anderen Endes eine Luftauslassöffnung auf. Der Luftauslass führt zu einem Mundstück. Zum Ausbringen des aktiven Kapselinhalts wird zunächst die Kapsel an üblicherweise zwei Stellen am längsseitigen Mantel geöffnet. In der Regel finden sich diese Öffnungen in der Nähe der beiden längsseitigen Enden der Kapsel. Wird nun in der Kapselkammer ein Luftstrom vom Lufteinlass zum Luftauslass erzeugt, führt dieser entlang der Längsachse der Kapsel und bewirkt dabei zweierlei: Zum einen wird die Kapsel durch den Luftstrom hauptsächlich entlang ihrer Längsachse bewegt. Diese Bewegung kann auch periodisch erfolgen, so dass die Kapsel vibriert. Zum anderen erzeugt die an den beiden Kapselöffnungen entlang strömende Luft vor den Kapselöffnungen gegenüber dem Kapselinneren einen Unterdruck, so dass das in der Kapsel befindliche Pulver vom Luftstrom mitgerissen und dabei vernebelt wird.

Die für solche Inhalatoren üblicherweise verwendeten Kapseln bestehen aus zwei becherartigen Teilen, die teleskopartig ineinander einsteckbar sind. Die äußere Form einer solchen zusammengesteckten Kapsel ist die eines geschlossenen Zylinders mit halbkugelförmigen Enden. Der Zylinder weist eine Längsachse und eine Querachse auf. Die Längsachse ist dabei diejenige Achse, die parallel zu den Erzeugenden des Zylindermantels liegt. Die Längsachse ist länger als die Querachse, so dass der Längsschnitt der Kapsel eine ovale, der Querschnitt ein kreisförmige Geometrie aufweist.

Üblicherweise bestehen die Kapseln für Pulverinhalativa aus Hartgelatine, sie können jedoch auch aus einem Kunststoffmaterial bestehen. In diesem Zusammenhang wird auf die EP 1100474 verwiesen.

Die DE 3345722 offenbart einen solchen, eingangs dieses Paragraphen (Stand der Technik) beschriebenen Inhalator aus zwei in radialer Richtung gegeneinander beweglichen Gehäuseelementen mit einer einzigen Kapselkammer. Die innere Oberfläche der hohlzylinderartigen Kapselkammer ist glatt.

Die WO 91/02558 offenbart einen anderen eingangs beschriebenen Inhalator, wobei jedoch anstelle einer einzigen Kapselkammer mehrere Kapselkammern ähnlich wie ein Revolvermagazin zusammengefasst sind. Die offenen Seiten dieses Magazins werden durch Wände begrenzt, wobei sich nur an einer Stelle in diesen Wänden der Lufteinlass bzw. Luftauslass befindet. Dieses Magazin ist drehbar so gelagert, dass eine Kapselkammer nur in einer bestimmten Position mit dem Lufteinlass, dem Luftauslass und den zum Öffnen der Kapsel notwendigen Schneideelementen in Verbindung steht.

Die EP 0911047 offenbart einen Inhalator mit einem a) nach oben hin offenen, becherförmigen Unterteil, b) einer Platte, welche die Öffnung des Unterteils bedeckt und senkrecht zu der eine Kapselkammer der oben beschriebenen Art ausgebildet ist, wobei an der Kapselkammer ein gegen eine Feder beweglicher Knopf vorgesehen ist, der zwei geschliffene Nadeln zum Öffnen der Kapsel aufweist, c) einem Oberteil mit einem Mundrohr, das - ein Pulveraerosol leiten könnend - mit der Kapselkammer verbunden ist und d) einem Deckel. Dabei sind die Elemente a), b) c) und d) durch eine gemeinsames Scharnierelement miteinander verbunden, so dass sie gegeneinander klappbar bewegt werden können. Daneben beschreibt diese Patentanmeldung einen Kapselhalter, wobei der Kapselhalter als Loch in der Platte b) ausgebildet sein kann und am Rand Rippen aufweist. Die Kapsel wird in diesen Kapselhalter zum Zweck der Bevorratung eingeklemmt.

Die FR-A-2 146 202 beschreibt ein Inhalationsgerät mit einer flachen zylindrischen Kammer, in der nur die Kapsel bewegt werden kann. Die an den Enden geöffnete Kapsel rotiert beim Inhalationsvorgang, durch tangential einströmende Luft angetrieben, um ihre Querachse.

In der US-A-4 069 819 ist ein Inhalator beschrieben, bei dem die Kapsel durch den Boden der Kapselkammer aufgestochen und während der Inhalation durch die in der Nähe des Bodens tangential eintretende Luft in Bewegung versetzt wird.
Die WO02/083220 zeigt einen verwandten Inhalator mit einer Kammer zur Aufnahme einer Kapsel, die ebenfalls durch den Boden der Kammer aufgestochen wird. An der inneren Oberfläche der Kammer ist mittig ein Ring angeordnet. Der obere, innen halbkugelförmig ausgebildete Teil der Kammer wird durch ein Inhalierstück gebildet und weist hierzu eine Vielzahl von Auslassöffnungen auf. Im unteren Bereich ist die Kammer zylinderförmig, wobei in der Wand eine Vielzahl an Lufteinlassschlitzen angeordnet ist.

### Beschreibung der Erfindung

Überraschend wurde nun festgestellt, dass sich die Wirkstoffausbringung mittels der aus dem Stand der Technik bekannten und oben beschriebenen Pulverinhalatoren verbessern lässt und eine gleichmäßigere Entleerung der Kapseln gewährleistet werden kann, wenn die Kapselkammer nicht nur von optimal größeren Dimensionen als die Kapsel ist, sondern wenn sie eine reliefartige innere Oberflächenstruktur aufweist, die die Kapsel bei der Vibration in der Kapselkammer zu führen vermag. Aus dem Stand der Technik sind nur Kapselkammern mit glatter innerer Oberfläche bekannt.

Durch die erfindungsgemäße Maßnahme wird bewirkt, dass die Kapsel mit dem aktiven Wirkstoff in der Kapselkammer hauptsächlich nur entlang ihrer Längsachse um eine kurze Strecke hin und her bewegbar ist und entlang ihrer Querachse nur einen geringen Spielraum aufweist und dabei gleichmäßig entleert wird. Zusätzlich werden auch die Berührungspunkte der Kapsel mit der inneren Kammeroberfläche minimiert und damit die Vibration vereinfacht.

Daher ist es eine Aufgabe der vorliegenden Erfindung Pulverinhalatoren mit Einwegkapseln als Wirkstoffreservoir zu schaffen, die die Wirkstoffausbringung verbessern.

Eine andere Aufgabe der Erfindung besteht darin, solche Pulverinhalatoren dahingehend zu verändern, dass eine gleichmäßige Entleerung verschiedener Kapseln gewährleistet wird.

Eine weitere Aufgabe besteht darin, einen derartigen Pulverinhalator mit einer Kapselkammer zu schaffen, in der sich eine Reservoirkapsel störungsfrei in Längsrichtung bewegen kann.

Eine weitere Aufgabe besteht darin, einen derartigen Pulverinhalator mit einer Kapselkammer zu schaffen, bei dem sich eine Reservoirkapsel, ohne die Längsbewegung der Kapsel zu stören, nur geringfügig quer bewegen kann.

Eine weitere Aufgabe besteht darin, Pulverinhalatoren mit Kapselkammern zu schaffen, bei denen die Kontaktfläche zwischen der inneren Oberfläche der Kammer und der Kapseloberfläche minimiert ist.

Eine weitere Aufgabe besteht darin, Pulverinhalatoren mit Kapselkammern zu schaffen, bei denen regelmäßig eine Entleerung von über 60%, bevorzugt über 90% erreicht wird.

### Detaillierte Beschreibung der Erfindung

Für die erfindungsgemäßen Pulverinhalatoren können die eingangs im Abschnitt Stand der Technik beschriebenen Inhalatoren, ausdrücklich die der DE 3345722, WO 91/02558 oder der EP 0911047 verwendet werden. D.h. die in dem Abschnitt Stand der Technik einleitend für die allgemein beschriebenen Pulverinhalatoren genannten Merkmale treffen mit Ausnahme der in Bezug auf die Gestaltung der Innenoberfläche der Kapselkammer genannten Merkmale ebenso auf den erfindungsgemäßen Pulverinhalator zu und sollen deshalb an dieser Stelle nicht noch einmal genannt werden. In den erfindungsgemäßen Inhalator können die im selben Abschnitt genannten Kapseln eingesetzt werden.

Die äußere Gestaltung der Kapselkammer spielt im Rahmen der vorliegenden Erfindung keine wesentliche Rolle. Die äußere Gestalt wird durch die Lage der Kapselkammer und deren eventuellen Bewegungen im Inhalator oder den Bewegungen von anderen Teilen des Inhalators um die Kapselkammer bestimmt.

Die innere Gestaltung der Kapselkammer ist erfindungsgemäß derart, dass sie einen nach zwei Seiten hin offenen Hohlraum zur Aufnahme einer Einwegkapsel für pharmazeutisch aktive Inhalativa aufweist. Bevorzugt liegen diese beiden Öffnungen an gegenüberliegenden Seiten oder in deren unmittelbarer Nähe. Die innere Form kann z.B. die eines bevorzugt gleichförmigen Zylinders oder Quaders sein. Bevorzugt ist die innere Gestalt an die Form eines Zylinders angelehnt.

Die Dimension der Kapselkammer ist an die der Kapsel angepasst. Zur Verdeutlichung werden deshalb im Folgenden einige Beispiele für typische Kapseldimensionen gegeben, die Rückschlüsse auf die Größe der Kapselkammer zulassen.
Gesamtlänge der geschlossenen Kapsel: 26,1 ±0,3 mm; 23,3 ±0,3 mm; 24,2 ±0,3 mm; 21,7 ±0,3 mm; 19,4 ±0,3 mm; 18,0 ±0,3 mm; 15,9 ±0,3 mm; 14,3 ±0,3 mm; 11,1 ±0,3 mm.
Äußerer Durchmesser der Kapselkörper: 9,55 mm; 8,18 mm; 7,36 mm; 7,34 mm; 6,63 mm; 6,07 mm; 5,57 mm; 5,05 mm; 4,68 mm.
Äußerer Durchmesser der Kapselkappen: 9,91 mm; 8,53 mm; 7,66 mm; 7,64 mm; 6,91 mm; 6,35 mm; 5,83 mm; 5,32 mm; 4,91 mm.
Die handelsüblichen Kapseln weisen die sogenannte Größe 3 auf, wie sie zumindest in Deutschland bekannt ist. Bei den beschriebenen Teleskopkapseln beträgt dabei der Durchmesser des Oberteils 5,83, der Durchmesser des Unterteils 5,57 mm.

Die erfindungsgemäße Kapselkammer weist eine innere Oberflächenstruktur auf, die einen davon eingeschlossenen inneren Hohlraum definiert. Unter innerem Hohlraum wird dabei der geometrische Raum verstanden, der die innere Oberfläche der Kapselkammer berührt, ohne dabei von optionalen Oberflächenstrukturen durchdrungen zu werden. Bevorzugt handelt es sich dabei um den entsprechenden Zylinderraum mit dem größten Durchmesser, der innerhalb der Oberflächenstrukturen aufspannbar ist.

Der innere Hohlraum weist einen Durchmesser auf, der 1,1- bis 2,5mal so groß ist wie der Kapseldurchmesser. Bevorzugt ist der Durchmesser 1,1- bis 2,2mal, insbesondere 1,2-bis 1,6mal so groß ist wie der Kapseldurchmesser.
Die Länge des inneren Hohlraums der Kapselkammer ist 1,02- bis 2mal so groß wie die Länge der Kapsel, vorzugsweise 1,04- bis 1,8-, insbesondere 1,1- bis 1,6mal so groß wie die Länge der Kapsel. Dabei soll der Durchmesser der Kammer kleiner sein als die Länge der Kapsel, so dass die Kapsel in der Kammer in der Längsrichtung gehalten ist und nicht auf die Seite kippen kann.

Wie bereits betont, weist die Kapselkammer zwei Öffnungen auf, einen Zugang für einströmende Luft und einen Luftausgang. Der Lufteinlass ist von geringerem Durchmesser als die Kapselkammer, so dass in diesem Bereich der Kapselkammer eine relativ hohe Strömungsgeschwindigkeit der Luft auftritt und ein Pulver in der Kapsel durch den Bernoulli-Effekt ausgebracht wird. Die Lufteintrittsöffnung wird zweckmäßig zentral im Boden der Kammer angeordnet.
An der Luftauslassseite kann eine Siebplatte oder eine andere Einrichtung, wie z.B. vorspringende Bauteile, ausgebildet sein, die verhindert, dass eine sich in der Kapselkammer bewegende Kapsel den Luftauslass blockieren kann oder dass eventuell entstandene Kapselbruchstücke in das Mundstück gesaugt werden.
Die Siebplatte kann dabei z.B. Teil eines trichterförmigen Verbindungsstückes sein, welches auf den Anfang des zum Mundstücks führenden Inhalationskanals so aufsteckbar ist, dass der Trichterrand mit der Siebplatte in eine Einsatzplatte eingreift, die den Boden des Mundstückes bildet. Die Siebplatte kann aber auch im Klemmsitz zwischen dem Trichterrand des Verbindungsstückes und einem Anschlag der Einsatzplatte austauschbar befestigt sein.

Es können auch mehrere Öffnungen als Auslassöffnung vorgesehen sein. Der für das Ausströmen der Luft aus der Kapselkammer zur Verfügung stehende Durchmesser ist zweckmäßig überall größer als die Lufteinlassöffnung, damit die mit dem Arzneimittel beladene Luft möglichst ungehindert ausströmen kann. Die Luftaustrittsöffnung wird zweckmäßigerweise zentral in der Decke der Kammer angeordnet, kann aber auch seitlich davon im Deckenbereich angeordnet sein.

Durch die Anordnung der beiden Öffnungen soll ein Luftstrom axial durch die Kapselkammer geführt werden.

Die Kapselkammer weist an wenigstens einer Stelle entlang ihrer Längsachse (bezogen auf den Innenraum der Kapselkammer) eine Öffnung für oder eine Verbindung mit eine(r) Schneidevorrichtung auf, die mit wenigstens zwei spitzen Nadeln oder Schneiden versehen ist, um eine in der Kapselkammer befindliche Kapsel aufzustechen oder aufzuschneiden. Bevorzugt handelt es sich um wenigstens zwei solcher Stellen, wobei durch jede dieser Stellen eine Schneide oder Nadel auf die Kapsel einwirken kann. Die Schneidevorrichtung ist gegen den Druck einer Feder ins Kammerinnere verschiebbar und wird über einen federnd gelagerten Betätigungsknopf bedient.
Die Spitzen bzw. Schneiden der Schneideeinrichtung sind bevorzugt so angeordnet, dass sie in den oberen Bereich bzw. unteren Bereich der Kapsel eindringen können. Da die Länge der Kapselkammer an die Länge der Arzneimittelkapseln angepasst ist, befinden sich die Schneiden bevorzugt in der Nähe des oberen bzw. unteren Endes der Kapselkammer. Die Seitenwand der Kapselkammer kann im Bereich ihres oberen und unteren Endes radiale Bohrungen oder längliche Schlitze aufweisen, die den Nadeln/Schneiden zugewandt sind und für die Durchführungen der Nadeln/Schneiden dienen. Die Dimension dieser Bohrungen/Schlitze ist dem Durchmesser der Nadeln oder Scheiden nachgebildet.

In einer bevorzugten Ausführungsform weist die Führung der Nadeln der Schneideeinrichtung eine Dichtungsplatte auf. Auf diese Weise wird die Dichtung zwischen der in Inhalationsposition befindlichen Kapselkammer und der Schneidevorrichtung verbessert. Für die federnde Lagerung der Dichtungsplatte kann die Feder verwendet werden, welche das Rückstellen der Betätigungstaste der Schneidevorrichtung bewirkt.

Schließlich ist in einer weiteren Ausführungsform ein Hebelsystem für die Betätigung der Schneideeinrichtung vorgesehen. Dieses Hebelsystem wird vorzugsweise von einer am Boden oder der Seite des Gehäuses des Inhalators angebrachten Betätigungstaste aus bestätigt. Das Hebelsystem kann aus einer Wippe und einem Kniehebel bestehen, wobei auf das eine Ende der Wippe die Betätigungstaste einwirkt, und das andere Ende der Wippe auf das eine Ende des Kniehebels drückt, wobei das an der Schneidevorrichtung befestigte andere Ende des Kniehebels die Schneidevorrichtung vorschiebt. Wippe und Kniehebel sind vorzugsweise um Achsen schwenkbar in Halterungen gelagert, die am Gehäuse befestigt sind.

Die Kapsel soll für den Inhalationsvorgang nahe ihren beiden Enden geöffnet werden. Dabei sollen die halbkugelförmigen Kappen der Kapsel in ihrem zentralen Bereich nicht beschädigt werden. Das ist deshalb wichtig, weil die Kapsel bzw. Kapselkappe eine Art Ventilfunktion ausübt. Aufgrund der Druckverhältnisse wird die Kapsel gegen die einströmende Luft an die Einlassöffnung gezogen und verschließt diese. Da der Benutzer weiter am Mundstück saugt, entsteht in der Kapselkammer ein Unterdruck, durch den die Kapsel mit der einströmenden Luft in Richtung auf den Luftauslass mitgerissen wird. Der nun am Lufteinlass entstehende Unterdruck bewirkt, dass die Kapsel erneut an die Einlassöffnung gezogen wird. Der ganze Vorgang wiederholt sich in rascher Folge, solange durch das Mundstück inhaliert wird, und versetzt die Kapsel in starke axiale Vibration.

Die aus dem Stand der Technik bekannten Kapselkammern für Pulverinhalatoren weisen eine konturenlose Innenoberfläche auf. Erfindungsgemäß weicht die Struktur der Innenoberfläche der vorliegenden Kapselkammer von einer solchen glatten Oberfläche ab.

Dies wird erreicht, indem auf der inneren Oberfläche der Kapselkammer, die im Zusammenhang mit der vorliegende Erfindung auch nur als Oberfläche der Kapselkammer bezeichnet wird, Abstandshalter zur Kapsel in Form von Erhebungen ausgebildet werden.

Die Abstandshalter sind dabei dergestalt, dass deren von der Oberfläche am weitest entfernten Punkte die Kapsel axial ausrichten und eine axiale Bewegung der Kapsel in der Kammer fördern. Gleichzeitig dürfen die Abstandshalter die Kapsel nicht seitlich einklemmen, so dass auch eine geringfügig seitliche Bewegung der Kapsel möglich ist.

Die Höhe der Abstandshalter (d.h. der Abstand Fußpunkt - Scheitelpunkt) beträgt bevorzugt 0,1 mm bis 5 mm, stärker bevorzugt 0,5 mm bis 2 mm.

Die Erhebungen weisen dabei bevorzugt jeweils gleiche Abstände zur Kapsel in der Kapselkammer auf.
Diese Abstände betragen bevorzugt 0,1 bis 1mm, stärker bevorzugt 0,2 bis 0,5 mm.

Die Erhebungen können dabei z.B. als Rippen mit abrupten Kanten, mit weichen, wellenförmigen Übergängen oder als Stifte ausgebildet sein. Kombinationen daraus sind auch möglich.
Die Spitzen oder Grate (Kanten) dieser Erhebungen weisen im bevorzugten Fall eine minimale Oberfläche auf.

Im Fall von Rippen können diese axial, d.h. parallel zur Längsachse der Kapsel in der Kammer, transversal, d.h. senkrecht zur Längsachse der Kapsel oder windschief zur Längsachse der Kapsel angeordnet sein. Dabei umfasst der Begriff "windschief auch schraubenförmig angeordnete Rippen.

Sind die Rippen axial angeordnet, weist die Kapselkammer zweckmäßigerweise wenigstens drei oder mehr solcher Rippen auf. Bevorzugt weist sie nicht mehr als neun, stärker bevorzugt nicht mehr als sechs derartiger Rippen auf. Die Länge der Rippen wird so gewählt, dass sie die Kapsel bei deren axialer Bewegung führen, ohne dass diese Bewegung blockiert werden kann. Bevorzugt erstrecken sich die Rippen über die gesamte Höhe der Kammer. In diesem Fall weisen die Rippen bevorzugt einen dreieckigen Querschnitt auf, wobei eine Spitze des Dreiecks von der inneren Kapselkammeroberfläche weg weist. Diese Ausführungsform hat den Vorteil, dass die Kapsel in der Kapselkammer bei ihrer axialen Bewegung ohne große Reibungsverluste geführt werden kann. Andere geometrische Ausgestaltungen der Rippen sind ebenfalls möglich, wie z.B. Rippen mit halbkreisförmigen oder rechteckigem Querschnitt etc.

Um ein Einklemmen der Kapsel in den axial angeordneten Rippen zu verhindern, können diese so angeordnet sein, so dass die Anordnung im Querschnitt nicht rotationssymmetrisch erscheint. D.h. bei beispielsweise drei Rippen stehen zwei der Rippen näher beieinander als zu der dritten.

Sind die Rippen tangential angeordnet, dann sollen alle zur Kapsel hinweisenden Kanten die Form eines gleichmäßigen Zylinders beschreiben. Bei solchen, um die Peripherie des Mantels der Kapselkammer laufenden Rippen ist es notwendig, dass die Abstände der Rippen so gewählt werden, dass die sich axial bewegende Kapsel, in ihrer Bewegung nicht von einer solchen Rippe behindert werden kann. In diesem Fall sind wenigstens zwei Rippen bevorzugt. Im Querschnitt weisen diese bevorzugt weiche Übergänge auf, d.h. es handelt sich um eine wellenartige Oberfläche. Diese Gestaltungsform hat den Vorteil, dass sich der Durchmesser der Kapselkammer entlang der Längsachse immer wieder ändert, so dass es bei axial durchströmender Luft entlang der Längsachse der Kapselkammer zu geringfügigen Druckunterschieden kommen kann, die das Entleeren der Kapsel fördern.

Eine weitere Ausführungsform der Kapselkammer weist eine Innenoberfläche mit einem wie eben beschriebenen transversalen Wellenverlauf auf, wobei auf dieser Oberfläche axial, d.h. senkrecht zu den Wellen Rippen ausgebildet sind. Dabei sind diese wiederum so ausgebildet, dass die äußeren Kanten der Rippen äquidistant zur zentralen Längsachse der Kapselkammer sind, die äußeren Kanten der Rippen also keine wellenförmige Oberfläche, sondern eine (parallel zur Längsachse) ungekrümmte Oberfläche besitzen.

Im Fall von stiftförmigen Erhebungen können diese entweder linear angeordnet sein und dabei gegebenenfalls die Rippen ersetzen, oder ihre Anordnung ist willkürlich. Die Stifte sind auf jeden Fall so ausgerichtet, dass die axiale Bewegung der Kapsel nicht gestört werden kann, sondern die Kapsel in dieser Bewegung geführt wird.

Die vorliegende Erfindung betrifft auch ein Ensemble von wenigstens zwei Kapselkammern, die konstruktiv miteinander verbunden sind. Ein solches Ensemble kann beispielsweise ein Magazin oder Revolvermagazin mit 3, 4, 5, 6, 7, 8, 9, 10, 11, 12, 13, 14, 15, 16, 17, 18, 19, 20, 21, 22, 23, 24, 25, 26, 27, 28, 29, 30 oder mehr Kapselkammern sein. Diese können kreisförmig, schraubenförmig oder spiralförmig aneinander gereiht sein, wobei jeweils die Kopfseiten der Kapselkammern in die gleiche Richtung weisen. Unter Kopfseite wird die Seite verstanden, in deren Nähe oder an der sich die Luftauslassöffnung befindet und an welcher der Kopf der Kapsel stößt (die schmale Seite der Kapsel). Bevorzugt sind in einem solchen Revolvermagazin die Kapselkammern kreis- oder spiralförmig aneinandergereiht.

Die erfindungsgemäße Kapselkammer kann in entsprechende Pulverinhalatoren eingebaut werden. Die so durch die erfindungsgemäße Kapselkammer modifizierten Pulverinhalatoren, die im einfachsten Fall aus der Kapselkammer, einer Lufteinlassöffnung, einer Luftauslassöffnung, die luftgängig mit einem Mundstück verbunden ist und gegebenenfalls eine Vorrichtung zum Öffnen der Kapseln bestehen, sind ebenfalls Gegenstand der vorliegenden Erfindung.
Dabei wird die in der Kammer mit dem Arzneistoff vermischte Luft durch das Mundstück dem Mund des Anwenders zugeleitet. Das Mundstück, das im allgemeinen röhrenförmig, gegebenenfalls etwas abgeflacht ist, kann axial oder auch in einem Winkel zur Achse der Kammer angeordnet oder seitlich zur Achse der Kammer versetzt sein.

Das Mundstück des Inhalators kann ähnlich wie eine Kappe ausgebildet sein, die auf das darunter liegende Bauteil (Unterteil) des Inhalators aufgesetzt wird, welches die Kapselkammer beinhaltet. Diese Kappe kann am Inhalatorgehäuserand um eine senkrecht zur Inhalatorlängsachse liegende Achse schwenkbar angelenkt sein. Mundstück und Unterteil des Inhalatorgehäuses können aber auch durch eine übliche Steckverbindung aneinander befestigt sein. Durch die Lösbarkeit bzw. Verschwenkbarkeit der beiden Teile ist jedenfalls der Zugang insgesamt, einerseits zur Kapselkammer und der Schneidevorrichtung im Gehäuseunterteil und andererseits zu den innen liegenden Teilen, wie die Siebplatte, das Gehäuseoberteil (der mundstückartigen Kappe) wesentlich vereinfacht.

Zum Austausch der verbrauchten Kapseln gegen frische wird in einer solchen Ausführungsform das Mundstück hochgeklappt oder die Steckverbindung zwischen Mundstück und Gehäuseunterteil gelöst. Die Kapselkammer ist dann frei zugänglich, so dass die entleerte Kapsel entnommen und eine gefüllte eingelegt werden kann. Sodann wird das Gerät zugeklappt bzw. zusammengesteckt.

Der erfindungsgemäße Inhalator ermöglicht gegenüber den aus dem Stand der Technik bekannten Geräten eine verlässlichere Ausbringung des Arzneimittels mit niedrigerer Standardabweichung. Gegenüber manchen der Geräte weist er auch den zusätzlichen Vorteil der besseren Desagglomeration auf. Die mikronisierten Arzneimittel in den Kapseln neigen nämlich zur Bildung von Agglomeraten. Diese Agglomerate sind therapeutisch unerwünscht, denn es wird eine möglichst feine Verteilung der Arzneimittel angestrebt. Bei der Benutzung des erfindungsgemäßen Inhalators werden die Agglomerate weitgehend zerstört.

Bevorzugte Inhalatoren sind solche wie sie eingangs als Ausführungsformen der DE 3345722, der WO 91/02558 oder der EP 0911047 beschrieben worden sind. Auf die in diesem Abschnitt genannten Merkmale wird an dieser Stelle noch einmal ausdrücklich hingewiesen. Besonders bevorzugt ist der Inhalator wie er oben im Zusammenhang mit der EP 0911047 beschrieben worden ist.

In solchen Inhalatoren kann nur eine der erfindungsgemäßen Kapselkammern ausgebildet sein, analog den Ausführungen zur DE 3345722 oder EP 0911047.
Die Kapselkammer kann aber auch Teil eines Kapselkammermagazins sein, wie es in der WO 91/02558 beschrieben wird.

Ein solcher Inhalator weist ein Revolvermagazin mit mehreren mit jeweils einer Kapsel bestückte, meist rohrförmige Kammern auf. Das Magazin ist an seinen beiden offenen Seiten durch jeweils eine Platte bedeckt, wobei die eine Platte die Lufteinlassöffnung und axial dazu die andere Platte die Luftauslassöffnung enthält. Da das Magazin innerhalb dieser Platten drehbar gelagert ist, kann jeweils eine der Kammern zwischen die beiden Öffnungen eingeschwenkt werden und somit einen Teil des Inhalationsluft-Durchgangskanals bilden. Nach Beendigung eines Inhalationsvorganges wird das Revolvermagazin weitergedreht, bis die nächstfolgende Kammer in den Luftdurchgangskanal kommt. Dabei kann z.B. eine der beiden Platten von dem Magazin getrennt werden, um die verbrauchten Kapseln aus den Kammern herauszunehmen oder das ganze Magazin kann z.B. zum Auffüllen herausgenommen werden.

In einer solchen Weiterbildung der Erfindung ist das Revolvermagazin lösbar im Inhalatorgehäuse angeordnet. Nach Verbrauch der im Revolvermagazin vorhandenen Kapseln kann somit das komplette Revolvermagazin ausgetauscht oder neu mit Kapseln gefüllt werden.

Das Inhalatorgehäuse kann einen exzentrisch angeordneten Stift besitzen, auf den das Revolvermagazin aufsteckbar ist.

Zur Fixierung der Position des Revolvermagazins kann man es mit den, den Kapselkammern jeweils zugeordneten Ausnehmungen für einen in dem Inhalatorgehäuse angeordneten federnd gelagerten Arretierbolzen versehen. Die Ausnehmungen sind so angeordnet, dass der Arretierbolzen nur dann dort einrastet, wenn eine der Kapselkammern sich genau zwischen Luftein- und -auslass befindet.

Damit kann sichergestellt werden, dass sich das Revolvermagazin während der Inhalation nicht verschiebt. Die federnde Lagerung des Arretierbolzens sollte hinsichtlich der Federkonstanten so gewählt werden, dass ein versehentliches Verdrehen des Revolvermagazins durch die Arretierung verhindert, andererseits bei stärkerer Krafteinwirkung das Revolvermagazin aus der Arretierung herausgedreht werden kann. Konische Ausgestaltungen des freien Endes des Arretierbolzens und entsprechend geformte Ausnehmungen wirken hierbei unterstützend.

Der Arretierbolzen ist vorzugsweise koaxial zum Luftdurchgangskanal unter der Kapselkammer angeordnet und weist eine Durchgangsbohrung auf, die gleichzeitig den bodenseitigen Lufteinlass bildet. Vorzugsweise ist der Arretierbolzen zentrisch im Inhalatorgehäuse angeordnet. Nach einer weiteren Ausgestaltung der Erfindung wird der Arretierbolzen durch eine Feder beaufschlagt, deren anderes Ende auf einem im Inhalatorgehäuse lösbar befestigten Stopfen aufliegt, der ebenfalls eine zentrale Durchgangsbohrung aufweist, die Teil des Luftdurchgangskanals ist.

In einer bevorzugten Ausführungsform sind die Ausnehmungen für den Eingriff des Arretierbolzens bodenseitig in der Bodenplatte des Magazins konzentrisch zu den Lufteintrittsbohrungen der Kapselkammern angeordnet und wie der Mantel eines mit der Basis nach außen gewandten flachen Kegelstumpfes gestaltet. Es handelt sich also bei diesen Ausnehmungen um konische bzw. trichterförmige Erweiterungen der Lufteintrittsbohrungen, wobei der erweiterte Bereich dem Arretierbolzen zugewandt ist. Die durch die Erweiterung entstehenden Schrägen entsprechen in etwa den Abschrägungen am Kopf des Arretierbolzens.

In einer bevorzugten Ausführungsform weisen diese Ausnehmungen an der Basis des Kegelstumpfmantels, aber noch in der Bodenplatte, eine umlaufende Anschlagskante auf, die als Verdrehsicherung bzw. Anschlag für den Kopf des Arretierbolzens dient, wenn dieser in die entsprechende Ausnehmung eingerastet ist. Aufgrund der besagten Anschlagskante kann man also bei eingerastetem Arretierbolzen das Magazin nicht weiterdrehen.

In einer anderen Ausgestaltung dieser Ausführungsform nimmt die besagte Anschlagskante nur einen Teil oder die Hälfte des Umfangs der konischen Ausnehmung, also der trichterförmigen Erweiterung, ein und ist so angeordnet, dass sie das Verdrehen des Magazins bei eingerastetem Arretierbolzen in einer Richtung sperrt, in der anderen Richtung aber zulässt, weil dort die schräge Wand der trichterförmigen Erweiterung der Ausnehmung glatt in die Außenseite der Bodenplatte übergeht.

In einer weiteren bevorzugten Ausführungsform weist nur eine der Ausnehmungen eine den ganzen Umfang der Ausnehmung einnehmende Anschlagskante auf, so dass in dieser Ausnehmung bei eingerastetem Arretierstift ein Verdrehen des Magazins nicht möglich ist. Diese Position betrachtet man dann als Endposition eines Magazins, in dem alle Kapseln verbraucht sind. Alle anderen Ausnehmungen weisen bei dieser Ausführungsform nur die einseitige, d.h. in einer Richtung wirkende Verdrehungssperre auf, so dass das Magazin immer nur in Richtung des Einschwenkens einer Kapselkammer mit einer unverbrauchten Kapsel gedreht werden kann, bis die zuvor geschilderte Endposition erreicht ist, in der die Arretierung vollständig ist. Der Benutzer weiß dann, dass das Magazin mit frischen Kapseln zu beschicken ist, wenn diese letzte Kapsel verbraucht ist.

In einer weiteren bevorzugten Ausführungsform kann am Arretierbolzen eine Zunge befestigt sein, die sich bis zu einem Anschlag an der Innenseite der Bedienungstaste der Schneidevorrichtung erstreckt, wenn der Arretierbolzen bei entnommenem Revolvermagazin seine obere Anschlagposition einnimmt. Die besagte Zunge wirkt in dieser Position als Sperre für die Schneideeinrichtung. Beim Einsetzen des Magazins wird der Arretierbolzen wieder nach unten gedrückt und damit die Sperre der Schneidevorrichtung beseitigt.

Die Betätigung der Schneidevorrichtung kann auch mit der Drehbewegung des Kapselmagazins gekoppelt werden, so dass mit einem Tastendruck zuerst eine Kapselkammer in die richtige Position gebracht wird und anschließend sofort die Schneidevorrichtung angreift.

Werden das Revolvermagazin und der an dieses angrenzende Teil des Inhalatorgehäuses n-eckig ausgestaltet, wobei n eine ganze, die Anzahl der Kapselkammern angebende Zahl ist, so lässt man vorteilhaft die Seitenflächen des Inhalatorgehäuseteils und des Revolvermagazins fluchten, wenn das Magazin in der richtigen Position ist. Man kann dann unmittelbar von außen feststellen, ob die Kammer in dem durch den Lufteinlass und den Luftauslass definierten Luftkanal liegt.

Als Alternative zu der erfindungsgemäßen Gestaltung der Innenoberfläche der Kapselkammer kann auch die äußere Oberfläche der Kapsel eine derartige Oberflächenstruktur aufweisen. Eine solche Alternative fällt aber nicht unter die vorliegende Erfindung. Unter dieser Alternative weisen Inhalationskapseln eine Oberflächenstruktur auf, die der Funktion nach der Oberflächenstruktur der Kapselkammer entspricht. So können beispielsweise die Kapseln axiale Rippen, Erhebungen, ringförmig umlaufende Ringe und dergleichen aufweisen. Die bezüglich der Gestaltung der Oberfläche der inneren Wandung der Kapselkammer beschriebene Oberflächenstruktur wird in diesem Fall auf die Außenoberfläche der Kapsel "projiziert". Wurden für die Kapselkammer beispielsweise axiale Rippen beschrieben, deren Querschnitt dreiecksförmig ist, mit einer der Seiten als Basisseite auf der Oberfläche und der Spitze zur Kapsel weisend, kann statt dessen die Kapsel auf ihrer Außenseite derartige axial angeordnete Rippen aufweisen, die nun ihrerseits in Richtung der Wandung der Kapselkammer weisen. Analoges gilt für andere Gestaltungsformen der Oberflächen.

Bei den erfindungsgemäßen Inhalationssystemen aus Kapsel und Inhalator weist die Kapselkammer die strukturierte Oberfläche auf. In einer Weiterbildung der Erfindung können beide Elemente, d.h. die Kapselkammer und die Kapsel, die strukturierte Oberfläche aufweisen.

Im Folgenden soll die Erfindung anhand von Zeichnungen näher beschrieben werden.
Figur 1 zeigt einen Querschnitt durch eine erfindungsgemäße zylindrische Kapselkammer mit 3 longitudinal angeordneten Rippen.
Figur 2 zeigt den Längsschnitt durch die Wand der erfindungsgemäßen zylindrischen Kapselkammer mit wellenartigen Rippen.
Figur 3 zeigt die Ausführungsform nach Figur 2 in Kombination mit der Ausführungsform nach Figur 1 als Längsschnitt durch die Wand der erfindungsgemäßen zylindrischen Kapselkammer.
Figur 4 zeigt eine erfindungsgemäße Kapselkammer mit einer schraubenförmig angeordneten Rippe.
Figur 5 zeigt eine erfindungsgemäße Kapselkammer mit quadratischem Querschnitt.
Figur 6 zeigt einen Inhalator mit der erfindungsgemäßen Kapselkammer.
Figuren 7 a bis d zeigen einen Pulverinhalator mit einem Revolvermagazin, welches mehrere erfindungsgemäße Kapselkammern enthält.
Figur 8 zeigt einen Pulverinhalator mit gegeneinander beweglichem Ober- und Unterteil.

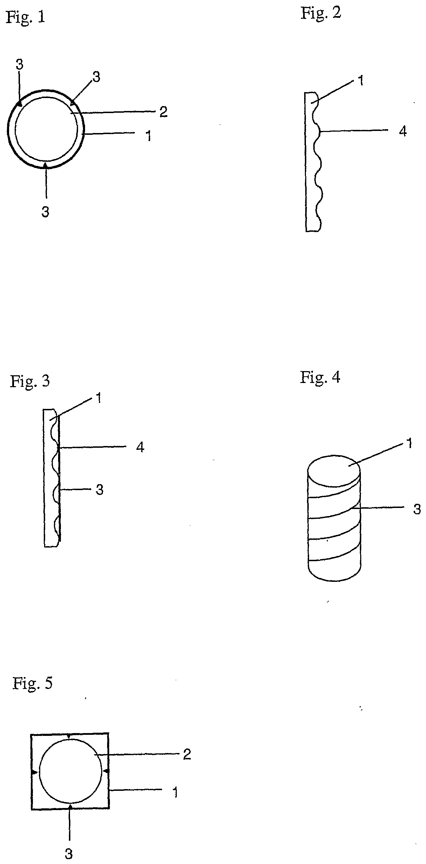

In Figur 1 ist der Querschnitt durch eine erfindungsgemäße zylindrische Kapselkammer (1) mit 3 longitudinal angeordneten Rippen (3) dargestellt. In der Kapselkammer (1) befindet sich eine Einweg-Inhalationskapsel (2).

Figur 2 zeigt den Längsschnitt durch die Wand der erfindungsgemäßen zylindrischen Kapselkammer (1) mit wellenartigen Rippen (4). Es ist nur eine Wandseite dargestellt.

Figur 3 gibt die Ausführungsform nach Figur 2 im Längsschnitt wieder, wobei senkrecht zu den wellenartigen Rippen (4) zusätzliche Rippen (3) in Längsrichtung nach Figur 1 dargestellt sind.

Figur 4 zeigt eine erfindungsgemäße Kapselkammern (1) mit schraubenförmig angeordneten Rippen (3).

Figur 5 zeigt eine erfindungsgemäße Kapselkammer (1) mit quadratischem Querschnitt und 4 Längsrippen (3).

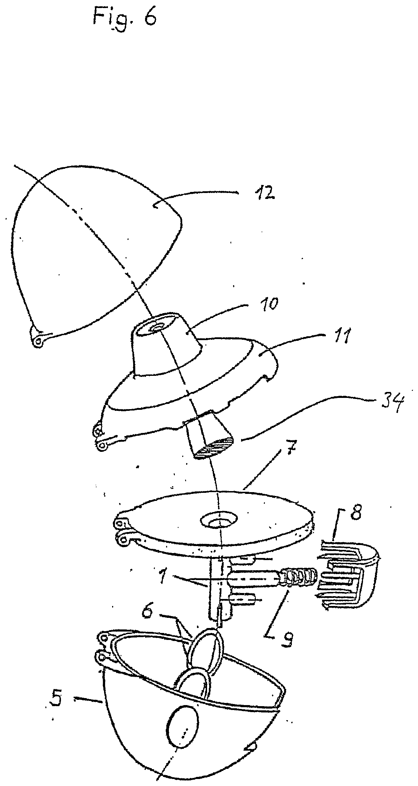

Aus Figur 6 ist zu entnehmen, wie ein Inhalator aufgebaut sein kann, in den eine erfindungsgemäße Kapselkammer integriert ist. In einem Unterteil (5) mit optional zwei Fenstern (6) befindet sich eine Platte (7), die mit der Kapselkammer (1) verbunden ist. Zum Öffnen der Kapseln in der Kapselkammer (1) dient der mit zwei speziell geschliffenen Nadeln versehene Knopf (8), der gegen den Druck der Feder (9) eingedrückt wird und dabei die Kapsel in der Kammer an zwei Stellen aufschneidet bzw. aufsticht. Beim Inhalieren durch das Gerät mittels des Mundrohrs (10), das mit dem Oberteil (11) verbunden ist, gelangt die Luft in das Unterteil (5) und von dort am unteren Ende in die erfindungsgemäße Kapselkammer (1). Das Gerät wird durch einen Deckel (12) verschlossen, der klappbar mit dem Unterteil (5), der Platte (7) und dem Oberteil (11) verbunden ist, so dass bei geschlossenem Deckel Staub nicht in das Gerät eindringen kann. In der Platte (7) können sich optional sacklochartige Kapselhalterungen befinden. Vorteilhaft ist eine Siebplatte (34), welche am unteren Ende des Mundrohrs (10) bzw. des zur Öffnung des Mundstücks führenden Inhalationskanals befestigt ist und im geschlossenen Zustand des Inhalators die Luftauslassöffnung der Kapselkammer (1) bedeckt. Nicht dargestellt sind optionale Schnapphäkchen an der zur Platte (7) hin orientierten Seite des Mundrohrs (10) bzw. des Oberteils (11), die in die Platte (7) einrasten können. In diesem Fall weist die Platte (7) entsprechend komplementäre Einrichtungen (Vertiefungen oder Löcher) auf. An der Platte (7) können ebenfalls z.B. seitlich Vorsprünge oder Schnapphäkchen ausgebildet sein, damit die Platte (7) in das Unterteil (5) einrasten kann. Die genannten Einrichtungen zum Einrasten des Mundstücks (10) bzw. des Oberteils (11) in die Platte (7) oder die der Platte (7) in das Unterteil (5) sind derart, dass die einzelnen Elemente einfach wieder getrennt werden können. Weiter kann an der Stelle des Deckels (12), die im geschlossenen Zustand über dem Knopf (8) liegt, eine Nase derart ausgebildet sein, dass diese in eine Vertiefung auf der Oberseite des Knopfes (8) eingreift und den Knopf (8) so blockiert, so dass der Knopf (8) im geschlossenen Zustand nicht gedrückt werden kann. Dadurch wird verhindert, dass bei vorzeitig in die Kapselkammer eingelegter Kapsel diese versehentlich gelocht werden kann.

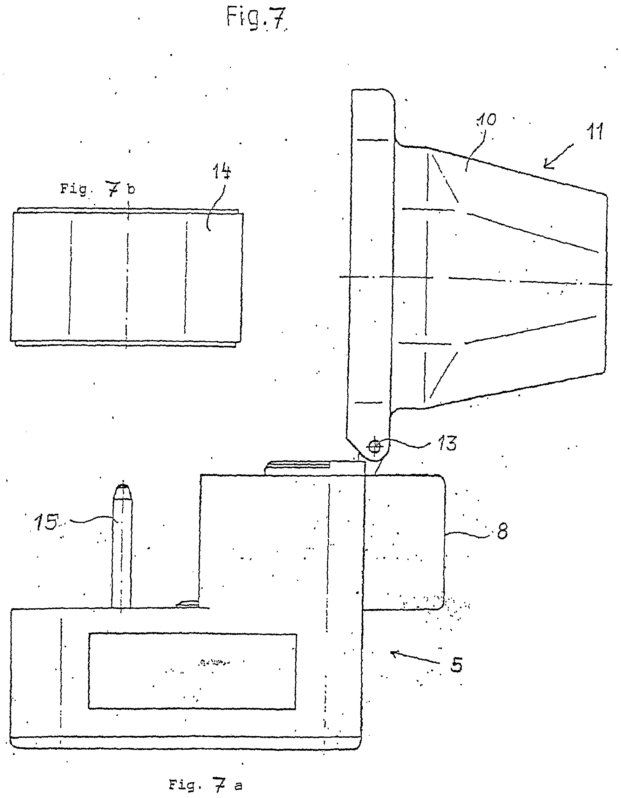

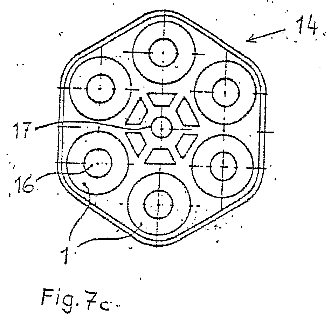

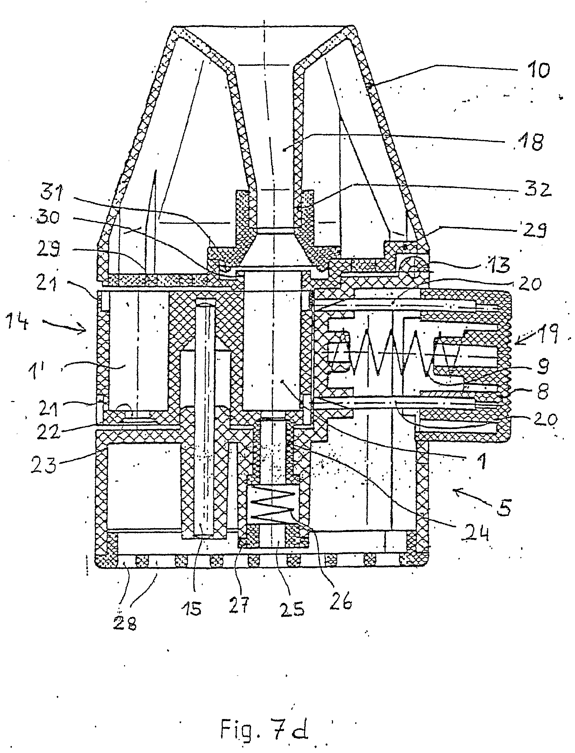

Figur 7: Wie aus Fig. 7a, 7b und 7c ersichtlich, besteht ein Inhalator mit Revolvermagazin im Wesentlichen aus einem Inhalatorgehäuse (5) mit einem Mundstück (10), das seitlich am oberen Rand des Inhalatorgehäuses (11) um eine Achse (13) schwenkbar angelenkt ist und einem Revolvermagazin (14) mit den erfindungsgemäßen Kammern (1) zur Aufnahme der Kapseln. Das Revolvermagazin (14) ist auf einen exzentrisch im Inhalatorgehäuse (5) angeordneten Stift (15) aufsteckbar. Nach Aufstecken des Revolvermagazins (14) wird das Mundstück (10) in seine Normalstellung - als Kappe auf dem Gehäuse - gebracht; der Inhalator ist funktionsfähig. Eine (nicht dargestellte) Kapsel kann nun durch Drücken des Knopfs (8) gelocht werden. Wie aus Figur 7c ersichtlich, besitzt das Revolvermagazin (14) in diesem Fall 6 Kammern (1) zur Aufnahme der nicht dargestellten Kapseln. Der Boden jeder Kammer (1) weist eine Lufteintrittsbohrung (16) auf. Ferner besitzt das Revolvermagazin (14) eine axiale Führung (17) für den Stift (15).

Wie aus Figur 7d ersichtlich, besitzt der Inhalator angrenzend an die unter dem Inhalationskanal (18) angeordnete Kammer (1) die Schneideeinrichtung (19), die über den Knopf (8) zu betätigen ist. Diese Schneideeinrichtung (19) weist zwei Nadeln (20) auf, die in den oberen bzw. unteren Teil der besagten Kammer (1) radial eingeführt werden können, wobei die Revolvermagazin-Außenwand zur leichteren Durchführung der Nadeln (20) an entsprechenden Stellen Durchbrüche oder geschwächte Bereiche (21) aufweist. Die Nadeln (20) dienen zum Öffnen der in der Kammer (1) befindlichen Kapsel in der Nähe von deren oberen bzw. unteren Ende. Das Revolvermagazin (14) besitzt ferner unterhalb der Bohrungen (22) konische Ausnehmungen (23), in die ein Arretierbolzen (24) einrasten kann, sobald die entsprechende der Kammern (1) koaxial mit dem Lufteinlass bzw. Inhalationskanal (18) des Inhalatorgehäuses ist. Der Arretierbolzen ist an seinem in die Ausnehmung (23) eingreifenden Ende ebenfalls konisch gestaltet. Am gegenüberliegenden Ende ist er durch eine Feder (26) beaufschlagt, die sich auf einem im Inhalatorgehäuse lösbar befestigten Stopfen (27) abstützt. Dieser Stopfen weist ebenso wie der Arretierbolzen eine zentrale Durchgangsbohrung auf, die als Lufteinlass (25) dient.

Zur Vorbereitung des Inhalators wird bei eingelegtem Revolvermagazin (14) eine der Kammern (1) durch Drehung des Revolvermagazins in eine Position gebracht, in der die bodenseitige Bohrung (22) bzw. die konische Ausnehmung (23) koaxial zur Lufteinlassöffnung (25) ausgerichtet ist. Die Einstellung der Kammer (1) wird durch Einrasten des Arretierbolzens (24) in die Ausnehmung (23) erleichtert. Nach dem Einrasten des Bolzens fluchten die Lufteintrittsöffnung (25) und die Bodenöffnung (22) der Kammer (1). Die Kapselkappe steht dabei auf der besagten Bodenöffnung (22) und verschließt diese. Durch Betätigung des Knopfs (18) gegen die Kraft einer Feder (9) werden die Schneiden (20) radial in Richtung auf die Kammer (1) bewegt, wobei sie zunächst die geschwächten Bereiche (21) durchstoßen bzw. in passende Öffnungen in der Seitenwand des Revolvermagazins eintreten und schließlich die Kapsel oben und unten nahe ihrem Ende öffnen. Dabei dürfen die halbkugelförmigen Kappen der Kapseln nicht zerstört werden.

Wird nunmehr Luft über das Mundstück (10) angesaugt, so versetzt die von den bodenseitigen Öffnungen (28) des Gehäuses (5) und dem Lufteinlass (25) her in die Kammer (1) einströmende Luft die Kapsel in heftige Vibration, wirbelt das Pulver in der Kapsel auf, vermischt sich damit und wird schließlich inhaliert. Das Mundstück (10) ist im allgemeinen röhrenförmig ausgebildet, kann jedoch auch der Mundform angepasst und abgeflacht sein. Ebenso sind in Abänderung der dargestellten Ausführungsform axiale oder in einem Winkel zur Achse der Kammer oder seitlich zur Kammerachse versetzt Mundstückanordnungen möglich.

Bodenseitig kann das Mundstück (10) mit einer im Wesentlichen geschlossenen Einsatzplatte (29) versehen sein. Diese Einsatzplatte (29) kann aber auch Durchbrechungen aufweisen. Ferner kann der Anfang des Inhalationskanals (18) mit einem Sieb bedeckt sein, welches verhindert, dass die Kapsel oder Kapselbruchstücke beim Inhalieren in den Inhalationskanal (18) im Mundstück gelangen. Alternativ hierzu können an der besagten Stelle Wandvorsprünge vorgesehen sein, welche die Kapsel zurückhalten. Die Siebplatte ist dann vorzugsweise im Zentrum der Einsatzplatte (29) angeordnet, vorteilhaft im Klemmsitz zwischen einem den Luftdurchlass umfassenden Anschlag (30) der Platte (29) und dem Rand eines trichterförmigen Verbindungsstückes (31), welches auf den Anfang (32) des Inhalationskanals (19.) so aufgesteckt ist, dass der Trichterrand der Einsatzplatte (29) zugewandt ist und mit dieser im Eingriff steht. Dort können auch die alternativ vorgesehenen Vorsprünge angeordnet sein.

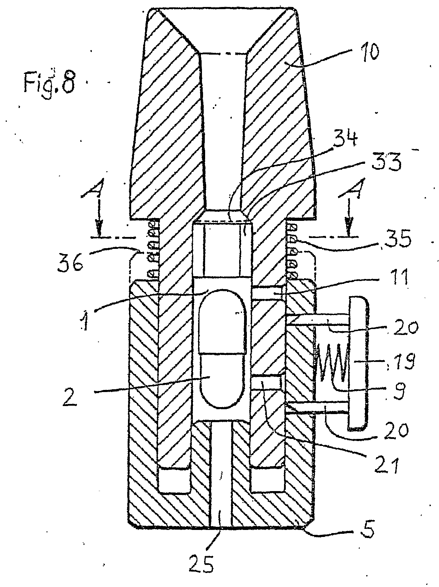

Die in Figur 8 wiedergegebene Ausführungsform des erfindungsgemäßen Inhalators besteht aus dem Unterteil (5) und dem Mundstück (10), die zusammengesteckt werden. Das Unterteil enthält den Lufteinlasskanal (25) der mit dem Lufteinlass in die Kapselkammer (1) verbunden ist. Die Schneidevorrichtung (19) wird durch ein Federelement (9) in ihrer Normalposition gehalten. Das Mundstück (10) enthält die Kapselkammer (1). Die erfindungsgemäße innere Oberflächenstruktur derselben ist nicht dargestellt, wie ebenfalls nicht in den Figuren 6 und 7. In die Verlängerung des Kapselraumes ragen Vorsprünge (33), die den Spielraum der Kapsel begrenzen. Eine Siebplatte (34) begrenzt die Kapselkammer und verhindert, dass z.B. Bruchstücke der Kapsel mit inhaliert werden können. Der Inhalator kann gegen den Druck eines Federelements (35) axial zusammengedrückt werden, wobei der obere Rand des Unterteils die Position (36) erreicht. In dieser Position können die Messer beziehungsweise Spitzen (20) der Schneidevorrichtung (19) durch die Öffnung (21) in die Kapselkammer (1) eindringen und die dort fixierte Kapsel öffnen.

Zur Benutzung des Inhalators nach Fig. 8 werden Unterteil (5) und Mundstück (10) auseinandergezogen, die Kapsel eingelegt und die beiden Inhalatorteile zusammengesteckt. Nach dem Zusammendrücken in Position (36) gegen das Federelement (35) wird die Schneidevorrichtung (19) betätigt und wieder losgelassen. Unter dem Druck des Federelementes (35) geht der Inhalator wieder in die in Figur 8 dargestellte Ausgangsposition über. Nun kann durch Einatmen durch das Mundstück (10) die Wirkstoffformulierung aus der nicht dargestellten Kapsel inhaliert werden.

## Patentansprüche

1. Inhalationssystem aus einer Einwegkapsel für pharmazeutisch aktive Inhalativa und einem Pulverinhalator mit einer Kapselkammer (1) in Form eines nach zwei Seiten hin offenen Hohlraums zur Aufnahme der Einwegkapsel, wobei der innere Hohlraum der Kapselkammer (1) einen Durchmesser, der 1,1- bis 2,5mal so groß wie der Kapseldurchmesser ist, und eine Länge aufweist, die 1,02- bis 2mal so groß wie die Länge der Kapsel ist, so dass die Einwegkapsel in der Kapselkammer (1) vibrieren kann oder bewegt werden kann, wenn ein Luftstrom durch die Kapselkammer (1) geführt wird, **dadurch gekennzeichnet,**
**dass** die innere Oberfläche der Kapselkammer (1) Abstandshalter für die Kapsel in Form von Erhebungen aufweist, so dass die Einwegkapsel im Wesentlichen nur eine Bewegung in Längsrichtung vollführen kann, wenn der Luftstrom durch die Kapselkammer (1) geführt wird.

2. Inhalationssystem nach Anspruch 1, **dadurch gekennzeichnet, dass** der Abstand des äußersten Punkts der Abstandhalter von der sie ausbildenden Fläche 0,1 mm bis 5 mm, bevorzugt 0,5 mm bis 2 mm beträgt.

3. Inhalationssystem nach einem der Ansprüche 1 oder 2, **dadurch gekennzeichnet, dass** der Abstand des Scheitels eines Abstandhalters zur Kapsel 0,1 bis 1mm, bevorzugt 0,2 bis 0,5 mm beträgt.

4. Inhalationssystem nach einem der Ansprüche 1 bis 3, **dadurch gekennzeichnet, dass** die Abstandhalter als axiale, transversale und/oder schraubenförmig verlaufende Rippen (3), als Punkte, Stifte oder wellenförmige Erhebungen ausgebildet sind.

5. Inhalationssystem nach einem der Ansprüche 1 bis 4, **dadurch gekennzeichnet, dass** die Abstandhalter als axiale Rippen (3) ausgebildet sind.

6. Inhalationssystem nach Anspruch 4, **dadurch gekennzeichnet, dass** nicht alle Rippen (3) den gleichen Abstand voneinander aufweisen.

7. Inhalationssystem nach einem der Ansprüche 1 bis 6, **dadurch gekennzeichnet, dass** die Rippen (3) im Querschnitt die Form eines Dreiecks haben.

8. Magazin für einen Pulverinhalator, wobei das Magazin aus wenigstens zwei Kapselkammern (1) mit Kapseln besteht, wobei die Kapselkammern (1) jeweils die Form eines nach zwei Seiten hin offenen Hohlraums haben, wobei der innere Hohlraum einen Durchmesser, der 1,1- bis 2,5mal so groß wie der Durchmesser der jeweiligen Kapsel ist, und eine Länge aufweist, die 1,02- bis 2mal so groß wie die Länge der jeweiligen Kapsel ist, so dass die Kapsel in der jeweiligen Kapselkammer (1) vibrieren kann oder bewegt werden kann, wenn ein Luftstrom durch die Kapselkammer (1) geführt wird,
**dadurch gekennzeichnet, dass** die innere Oberfläche der Kapselkammern (1) Abstandshalter für die Kapseln in Form von Erhebungen aufweist, so dass die Kapseln im Wesentlichen nur eine Bewegung in Längsrichtung vollführen können, wenn der Luftstrom durch die jeweilige Kapselkammer (1) geführt wird.

9. Magazin nach Anspruch 8, **dadurch gekennzeichnet, dass** das Magazin ein Revolvermagazin (14) ist.

10. Magazin nach Anspruch 9, **dadurch gekennzeichnet, dass** das Magazin ein Revolvermagazin (14) ist, das 2, 3, 4, 5, 6, 7, 8, 9, 10, 11, 12, 13, 14, 15, 16, 17, 18, 19, 20, 21, 22, 23, 24, 25, 26, 27, 28, 29 oder 30 Kapselkammern (1) aufweist, die kreis- oder schraubenförmig so aneinandergereiht sind, dass zwei benachbarte Kapselkammern (1) parallel zueinander ausgerichtet sind, wobei die bodenseitigen und die kopfseitigen Enden jeweils in einer Fläche liegen.

11. Pulverinhalator bestehend aus einem oberen Gehäuseteil (11), welches ein mit der Luftauslassöffnung in Verbindung stehendes Mundstück aufweist und einem unteren Gehäuseteil (5) mit einem Magazin nach einem der Ansprüche 8 oder 9, wobei die Kapselkammern eine Lufteinlassöffnung und eine Luftauslassöffnung aufweisen.

12. Pulverinhalator nach Anspruch 11, **dadurch gekennzeichnet, dass** der Pulverinhalator eine Schneidevorrichtung aufweist, die mit wenigstens zwei spitzen Nadeln und/oder Schneiden bestückt ist, wobei die Nadeln und/oder Schneiden durch Öffnungen in die Kapselkammern (1) eingeführt werden können.

13. Pulverinhalator nach Anspruch 11 oder 12, **gekennzeichnet durch** a) ein nach oben hin offenes, becherförmiges Unterteil (5), b) eine Platte (7), welche die Öffnung des Unterteils (5) bedeckt und senkrecht zu der eine Kapselkammer (1) der oben beschriebenen Art ausgebildet ist, an der Kapselkammer (1) ein gegen eine Feder beweglicher Knopf (8) vorgesehen ist, der zwei geschliffene Nadeln oder Schneiden zum Öffnen der Kapsel aufweist, c) ein Oberteil (11) mit einem Mundrohr (10), das ein Pulveraerosol leiten könnend mit der Kapselkammer (1) verbunden ist und d) ein Deckel (12), wobei die Elemente a), b) c), und d) durch ein gemeinsames Scharnierelement miteinander verbunden sind, so dass sie gegeneinander klappbar bewegt werden können.

## Claims

1. Inhalation system from a disposable capsule for pharmaceutically active inhalable compositions and a powder inhaler with a capsule chamber (1) in the form of a cavity open on two sides for accommodating the disposable capsule, wherein the inner cavity of the capsule chamber (1) has a diameter which is 1.1 to 2.5 times as great as the capsule diameter and a length which is 1.02 to 2 times as great as the length of the capsule, so that the disposable capsule in the capsule chamber (1) can vibrate or can be moved when an airstream is passed through the capsule chamber (1), **characterised in that** the inner surface of the capsule chamber (1) comprises spacers for the capsule in the form of raised portions, so that the disposable capsule can essentially perform only a movement in a longitudinal direction when the airstream passes through the capsule chamber (1).

2. Inhalation system according to claim 1, **characterised in that** the spacing of the outermost point of the spacers from the surface on which they are formed is 0.1 mm to 5 mm, preferably 0.5 mm to 2 mm.

3. Inhalation system according to one of claims 1 or 2, **characterised in that** the spacing of the apex of a spacer from the capsule is 0.1 to 1 mm, preferably 0.2 to 0.5 mm.

4. Inhalation system according to one of claims 1 to 3, **characterised in that** the spacers are constructed as axial, transverse and/or helically extending ribs (3), as points, pins or undulating raised portions.

5. Inhalation system according to one of claims 1 to 4, **characterised in that** the spacers are constructed as axial ribs (3).

6. Inhalation system according to claim 4, **characterised in that** the ribs (3) are not all at the same spacing from one another.

7. Inhalation system according to one of claims 1 to 6, **characterised in that** the ribs (3) are triangular in cross section.

8. Magazine for a powder inhaler, wherein the magazine consists of at least two capsule chambers (1) with capsules, the capsule chambers (1) each being in the form of a cavity open on two sides, the inner cavity having a diameter which is 1.1 to 2.5 times as great as the diameter of the respective capsule and a length which is 1.02 to 2 times as great as the length of the respective capsule, so that the capsule in the capsule chamber (1) can vibrate or can be moved when an airstream is passed through the capsule chamber (1), **characterised in that** the inner surface of the capsule chamber (1) comprises spacers for the capsules in the form of raised portions, so that the capsules can essentially perform only a movement in a longitudinal direction when the airstream passes through the respective capsule chamber (1).

9. Magazine according to claim 8, **characterised in that** the magazine is a revolver magazine (14).

10. Magazine according to claim 9, **characterised in that** the magazine is a revolver magazine (14) which has 2, 3, 4, 5, 6, 7, 8, 9, 10, 11, 12, 13, 14, 15, 16, 17, 18, 19, 20, 21, 22, 23, 24, 25, 26, 27, 28, 29 or 30 capsule chambers (1) arranged in a circular or spiral configuration with one another such that two adjacent capsule chambers (1) are aligned parallel to one another, the base and top ends each being located in a plane.

11. Powder inhaler consisting of an upper housing part (11) which has a mouthpiece connected to the air outlet opening and a lower housing part (5) with a magazine according to one of claims 8 or 9, wherein the capsule chambers have an air inlet opening and an air outlet opening.

12. Powder inhaler according to claim 11, **characterised in that** the powder inhaler has a cutting device which is fitted with at least two sharp spikes and/or cutters, the spikes and/or cutters being capable of being inserted through openings into the capsule chambers (1).

13. Powder inhaler according to claim 11 or 12, **characterised by** a) a cup-shaped lower part (5) open at the top, b) a plate (7) which covers the opening of the lower part (5) and perpendicularly to which is formed the capsule chamber (1) of the type described above, a button (8) movable counter to a spring is provided on the capsule chamber (1), comprising two sharp spikes or cutters for opening the capsule, c) an upper part (11) with a mouth tube (10) which is connected to the capsule chamber (1) so as to be able to convey a powder aerosol and d) a lid (12), the elements a), b) c) and d) being joined together by a common hinge element such that they can be flipped back and forth relative to one another.

## Revendications

1. Système d'inhalation, constitué d'une capsule jetable pour des produits à inhaler pharmaceutiquement actifs et d'un inhalateur de poudre, avec un compartiment à capsule (1) sous forme d'une cavité, ouverte vers deux côtés, pour recevoir la capsule jetable, dans lequel la cavité intérieure du compartiment à capsule (1) présente un diamètre qui est 1,1 à 2,5 fois plus grand que le diamètre de la capsule, et une longueur qui est 1,02 à 2 fois plus grande que la longueur de la capsule de sorte que la capsule jetable puisse, dans le compartiment à capsule (1), vibrer ou être mise en mouvement quand un courant d'air est guidé à travers le compartiment à capsule (1),
**caractérisé en ce**
**que** la surface intérieure du compartiment à capsule (1) présente des entretoises pour la capsule sous forme de surélévations de sorte que la capsule jetable ne puisse sensiblement qu'exécuter un mouvement dans la direction longitudinale quand le courant d'air est guidé à travers le compartiment à capsule (1).

2. Système d'inhalation selon la revendication 1, **caractérisé en ce que** la distance du point le plus extérieur des entretoises, depuis le plan qui les forme, est de 0,1 mm à 5 mm, de préférence de 0,5 mm à 2 mm.

3. Système d'inhalation selon l'une des revendications 1 ou 2, **caractérisé en ce que** la distance du sommet d'une entretoise à la capsule est de 0,1 à 1 mm, de préférence de 0,2 à 0,5 mm.

4. Système d'inhalation selon l'une des revendications 1 à 3, **caractérisé en ce que** les entretoises sont conçues comme des nervures (3) axiales, transversales et/ou s'étendant hélicoïdalement, comme des points, des tiges ou des surélévations ondulées.

5. Système d'inhalation selon l'une des revendications 1 à 4, **caractérisé en ce que** les entretoises sont conçues comme des nervures (3) axiales.

6. Système d'inhalation selon la revendication 4, **caractérisé en ce que** toutes les nervures (3) ne présentent pas la même distance les unes aux autres.

7. Système d'inhalation selon l'une des revendications 1 à 6, **caractérisé en ce que** les nervures (3) ont en section transversale la forme d'un triangle.

8. Chargeur pour un inhalateur de poudre, dans lequel le chargeur comprend au moins deux compartiments à capsule (1) avec des capsules, dans lequel les compartiments à capsule (1) ont chacun la forme d'une cavité ouverte vers deux côtés, dans lequel la cavité intérieure présente un diamètre qui est 1,1 à 2,5 fois plus grand que le diamètre de la capsule respective, et une longueur qui est 1,02 à 2 fois plus grande que la longueur de la capsule respective de sorte que la capsule puisse, dans le compartiment à capsule (1) respectif, vibrer ou être mise en mouvement quand un courant d'air est guidé à travers le compartiment à capsule (1),
**caractérisé en ce que** la surface intérieure des compartiments à capsule (1) présente des entretoises pour les capsules sous forme de surélévations de sorte que les capsules ne puissent sensiblement qu'exécuter un mouvement dans la direction longitudinale quand le courant d'air est guidé à travers le compartiment à capsule (1) respectif.

9. Chargeur selon la revendication 8, **caractérisé en ce que** le chargeur est un chargeur rotatif (14).

10. Chargeur selon la revendication 9, **caractérisé en ce que** le chargeur est un chargeur rotatif (14), qui présente 2, 3, 4, 5, 6, 7, 8, 9, 10, 11, 12, 13, 14, 15, 16, 17, 18, 19, 20, 21, 22, 23, 24, 25, 26, 27, 28, 29 ou 30 compartiments à capsule (1), qui se succèdent sous une forme circulaire ou hélicoïdale de sorte que deux compartiments à capsule (1) voisins soient orientés parallèlement l'un à l'autre, dans lequel les extrémités côté fond et côté tête se trouvent chacune dans un plan.

11. Inhalateur de poudre constitué d'une partie de boîtier supérieure (11), qui présente un embout buccal relié à l'orifice de sortie d'air, et d'une partie de boîtier inférieure (5), avec un chargeur selon l'une des revendications 8 ou 9, dans lequel les compartiments à capsule présentent un orifice d'entrée d'air et un orifice de sortie d'air.

12. Inhalateur de poudre selon la revendication 11, **caractérisé en ce que** l'inhalateur de poudre présente un dispositif de coupe, qui est garni d'au moins deux aiguilles ou lames de coupe aiguisées, dans lequel les aiguilles et/ou les lames de coupe peuvent être insérées par des ouvertures dans les compartiments à capsule (1).

13. Inhalateur de poudre selon la revendication 11 ou 12, **caractérisé par** a) une partie inférieure (5) en forme de coupe, ouverte vers le haut, b) une plaque (7), qui recouvre l'ouverture de la partie inférieure (5) et qui est conçue perpendiculairement à l'un des compartiments à capsule (1) du type décrit ci-dessus, contre le compartiment à capsule (1) est prévu un bouton (8), mobile contre un ressort, qui présente deux aiguilles ou lames de coupe aiguisées pour ouvrir les capsules, c) une partie supérieure (11), avec un tube buccal (10), qui est reliée au compartiment à capsule (1) en pouvant diriger un aérosol en poudre, et d) un couvercle (12), dans lequel les éléments a), b), c) et d) sont reliés les uns aux autres par un élément à charnière commun de sorte qu'ils puissent être déplacés en pouvant être rabattus les uns contre les autres.
